# EUROPEAN PATENT APPLICATION

(11) **EP 1 889 603 A2**
(43) Date of publication of application: **20.02.2008**
(21) Application number: 07014661.8
(22) Date of filing: 26.07.2007
(51) Int. Cl.: A61K 8/49, A61K 8/58, A61K 8/67, A61Q 5/10

(54) **Hair dye**

(30) Priority: 28.07.2006 JP 2006205509; 31.05.2007 JP 2007144415
(71) Applicant: FUJIFILM Corporation, Minato-ku Tokyo (JP)
(72) Inventor: Jimbo, Yoshihiro, Minami-ashigara-shi Kanagawa 250-0193 (JP); Takahashi, Eri, Ashigarakami-gun Kanagawa 258-8577 (JP); Shimada, Yasuhiro, Ashigarakami-gun Kanagawa 258-8538 (JP); Sato, Kozo, Ashigarakami-gun Kanagawa 258-8577 (JP)
(74) Representative: HOFFMANN EITLE

(57) **Abstract**

A hair dye comprising, together or separately, (a) a carboxylic acid silver salt and/or a sulfonic acid silver salt, and (b) a reductone, e.g., reductones of the following formula (I) wherein R represents hydrogen atom or hydroxyl group; and p represents an integer of 1 to 4. There is provided a hair dye having superior hair dyeing property and hair dyeing reproducibility, with superior toughness of dyed hair condition against shampoo, conditioner or light and causing reduced damage to hair.

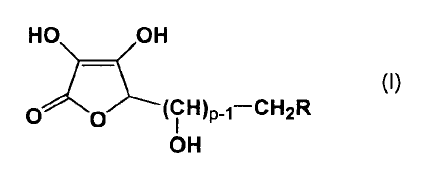

## Description

### Technical Field

The present invention relates to a hair dye. More specifically, the present invention relates to a hair dye which contains a silver salt and a reductone, together or separately, and has superior hair dyeing property and hair dyeing fastness, whilst causes reduced damage to hair.

### Background Art

Hair dyes using an oxidation dye have been so far widely known. These hair dyes consist of a first solution containing an oxidation dye intermediate such as para-phenylenediamine or 2,5-diaminotoluene, and an alkali such as ammonia, and a second solution containing an oxidizing agent such as hydrogen peroxide. It is known that the hair dyes using an oxidation dye have high hair dyeing power and fastness of dyed hair condition, as well as less color fade with shampoo, conditioner or light, because an oxidation dye intermediate penetrates into hair to dye the hair through oxidative polymerization. Further, the dyes also have characteristic feature that they are capable of simultaneously bleaching and dyeing hair by using aqueous hydrogen peroxide in combination, thereby hair dyeing in various color tones is achievable. owever, hair dyes using an oxidation dye have a problem that the alkali such as ammonia and the oxidizing agent used in the hair dyeing treatment cause damage to hair, resulting in hair with poor gloss and moisture and with unnatural touch. They also have a problem of unpleasant odor during hair dyeing when ammonia is used as the alkali Further, para-phenylenediamine, 2,5-diaminotoluene, and the like are usually used for the oxidation dyes, and it is pointed out that these substances show skin irritancy. Furthermore, although hair dyes using an oxidation dye can dye hair in various color tones, these color tones are not satisfactorily bright.

As hair dyes for dyeing hair into bright color tones, direct dyes have been proposed. Although the direct dyes are characterized to be capable of brightly dyeing hair, they have problems of poor fastness of dyed hair condition against shampoo, conditioner or light, as well as color fade, dull color, and the like. Further, hair dyes using a direct dye need to use an alkali such as ammonia to improve penetration of the dye into hair, and therefore they have problems of unpleasant odor during hair dyeing, and damage to hair due to the hair dyeing treatment. Furthermore, they also have problems of unnatural gloss, moisture and touch of hair after hair dyeing in the same manner as the use of the oxidation dyes.

Hair dyes using a silver salt are also known (Patent documents 1 to 6). These hair dyes utilize the photosensitivity of silver salts, and hair dyeing is attained by applying a silver salt to hair and then reducing the silver salt with natural or artificial light to develop a color. However, they have problems that they require much time for color development, and provide poor developed color density. Further, hair dyes using a silver salt and a reducing agent are also known (Patent documents 7 to 11). Although these hair dyes give quick color development, they have problems of poor developed color density, low reproducibility of hair dyeing density, and the like. Furthermore, they also have problems of unpleasant odor during hair dyeing, and damage to hair due to the hair dyeing treatment when an alkali such as ammonia is used to enhance penetration of silver ions into hair, and problems of unnatural gloss, moisture and touch of hair after hair dyeing. Moreover, they also have a problem of stains by coloring when a silver salt adhered to the skin or clothes.
Patent document 1: Japanese Patent Unexamined Publication (Kokai) No. 2005-60354
Patent document 2: Japanese Patent Unexamined Publication No. 2005-53889
Patent document 3: Japanese Patent Unexamined Publication No. 2004-99502
Patent document 4: Japanese Patent Unexamined Publication No. 2002-348221
Patent document 5: German Patent No. DE2714753
Patent document 6: International Patent Publication WO2006/011228
Patent document 7: Japanese Patent Publication (Kokoku) No. 7-116016
Patent document 8: Japanese Patent Unexamined Publication No. 4-187625
Patent document 9: Japanese Patent Unexamined Publication No. 48-18438
Patent document 10: German Patent No. DE2806603
Patent document 11: Japanese Patent No. 3759120

### Disclosure of the Invention

### Object to be Achieved by the Invention

An object of the present invention is to provide a hair dye which solves at least some of the aforementioned problems. More specifically, the object of the present invention is to provide a hair dye which have superior hair dyeing property and hair dyeing reproducibility with superior fastness of dyed hair condition against shampoo, conditioner or light, and causes reduced damage to hair and no stain on the skin and clothes.

### Means for Achieving the Object

The inventors of the present invention conducted various researches to achieve the foregoing object. As a result, they found that a hair dye having superior hair dyeing property and hair dyeing reproducibility, with superior fastness of dyed hair condition and causing reduced damage to hair and no stain on the skin and clothes was successfully provided by using a carboxylic acid silver salt and a sulfonic acid silver salt, and a reductone in combination. The present invention was accomplished on the basis of the aforementioned finding.

The present invention thus provides a hair dye comprising, together or separately:
(a) a carboxylic acid silver salt and/or a sulfonic acid silver salt, and
(b) a reductone,

According to preferred embodiments, the present invention provides the aforementioned hair dye, wherein the reductone of the aforementioned component (b) is a compound represented by the following general formula (I), or a salt thereof : 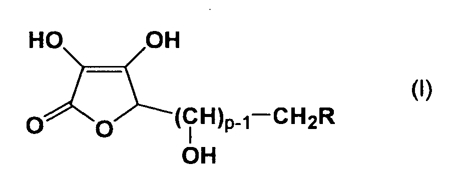 wherein R represents hydrogen atom or hydroxyl group; and p represents an integer of 1 to 4 ; the aforementioned hair dye, wherein the reductone is ascorbic acid or a salt thereof ; the aforementioned hair dye, wherein the water-soluble silver salt consists of one or more kinds of silver salts selected from the group consisting of a silver carboxylate, a silver sulfonate and silver nitrate; the aforementioned hair dye, wherein the water-soluble silver salt of the aforementioned component (a) is a silver salt selected from the group consisting of a carboxylic acid silver salt represented by the following general formula (II): R¹-(COOAg)ₙ wherein R¹ represents an n-valent organic group; and n represents an integer of 1 to 3, and a sulfonic acid silver salt represented by the following general formula (III): R²-(SO₃Ag)ₙ wherein R² represents an n-valent organic group, and n represents an integer of 1 to 3; and the aforementioned hair dye, wherein the carboxylic acid silver salt and/or the sulfonic acid silver salt of the aforementioned component (a) consists of one or more kinds of silver salts selected from the group consisting of silver acetate, silver propionate and silver lactate.

As a preferred embodiment of the aforementioned hair dye, there is provided the hair dye comprising a combination of a solution containing a carboxylic acid silver salt and/or a sulfonic acid silver salt of the aforementioned component (a) (solution A) and a solution containing a reductone of the aforementioned component (b) (solution B). As a more preferred embodiment, there is provided the hair dye in the form of a kit comprising a container filled with the aforementioned solution A and a container filled with the aforementioned solution B. According to still more preferred embodiments, there are provided the aforementioned hair dye, wherein content of the carboxylic acid silver salt and/or the sulfonic acid silver salt in the aforementioned solution A is 0.03 to 10% by mass in terms of mass of silver; the aforementioned hair dye, wherein content of the reductone, preferably a compound represented by the general formula (I) or a salt thereof, in the aforementioned solution B is 0.1 to 10% by mass; and the aforementioned hair dye, wherein pH of the solution A and/or the solution B is 7 or lower.

From another aspect, the present invention also provides a hair dyeing method comprising the step of applying the hair dye containing a carboxylic acid silver salt and/or a sulfonic acid silver salt of the aforementioned component (a) and a reductone of the aforementioned component (b), together or separately, to hair, preferably to human hair. According to preferred embodiments of this invention, there are provided the method comprising the step of mixing a solution containing a carboxylic acid silver salt and/or a sulfonic acid silver salt of the aforementioned component (a) (solution A) and a solution containing a reductone, preferably a compound represented by the general formula (I) or a salt thereof, of the aforementioned component (b) (solution B) upon use and applying the mixture to hair; the method comprising the step of applying the aforementioned solution A and then applying the aforementioned solution B to hair; and the method comprising the step of applying the aforementioned solution B and then applying the aforementioned solution A to hair. According to a more preferred embodiment, there is provided the method, wherein the aforementioned solution B contains one or more kinds of reducing agents other than the reductone.

The hair dye of the present invention is characterized to have superior hair dyeing property and hair dyeing reproducibility, with high fastness of dyed hair condition against shampoo, conditioner or light, and causes reduced damage to hair and no stain on the skin and clothes. Further, according to a particularly preferred embodiment of the present invention, the hair dye has characteristic feature of no unpleasant odor and no degradation of touch of hair after hair dyeing in addition to the aforementioned characteristic features.

### Best Mode for Carrying out the Invention

Reductone used as the component (b) of the hair dye of the present invention is a generic name of compounds having carbonyl group adjacent to ethylene diol (R"-C(OH)=C(OH)-COR''') and analogues thereof, and reductones of endiol type, enaminol type, endiamine type, thiol-enol type and enamine-thiol type are generally known. As the component (b) of the hair dye of the present invention, arbitrary reductones can be used so far that the reductone can reduce a carboxylic acid silver salt and/or a sulfonic acid silver salt. Typical examples of the reductones include ascorbic acid, reductonic acid, and the like, and the reductones described in U.S. Patent No. 2,688,549, Japanese Patent Unexamined Publication No. 62-237443, and the like can be exemplified. Synthetic methods for these reductones are also well known, and they are described in, for example, Nomura D. and Ohmura H., "Chemistry of Reductones" (Uchida Rokakuho Shinsha, 1969).

Specific examples of preferred reductones used as the component (b) of the hair dye of the present invention will be mentioned below. However, the reductones usable for the present invention are not limited to the following examples.

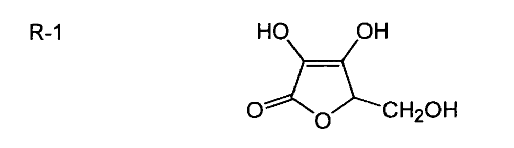

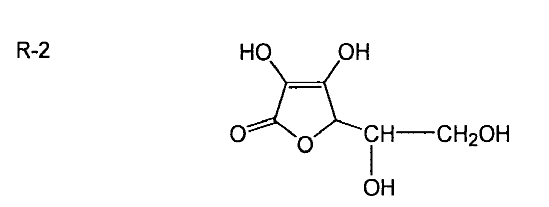

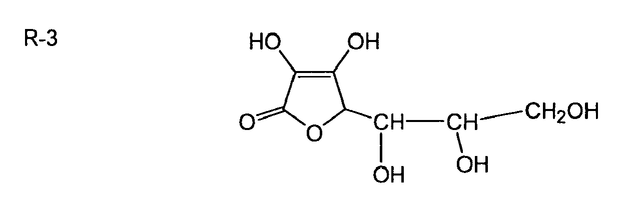

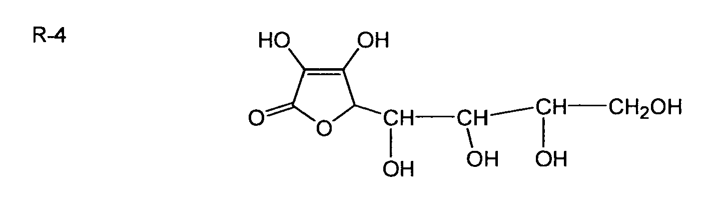

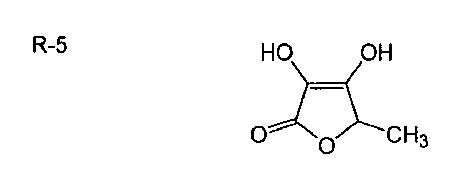

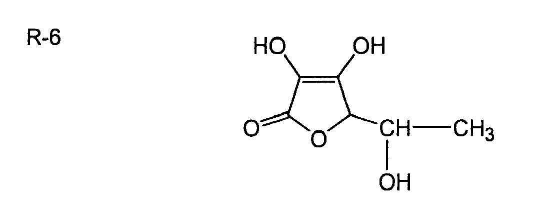

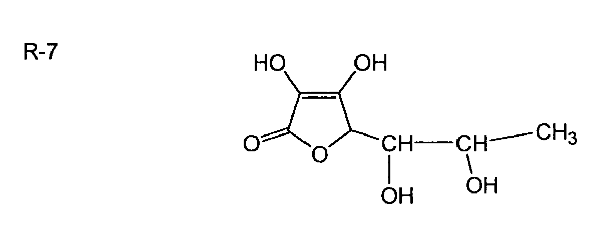

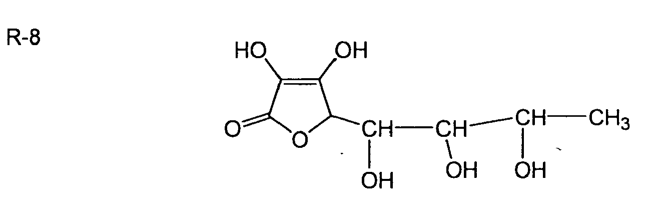

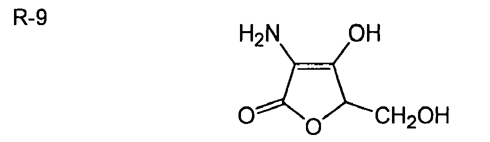

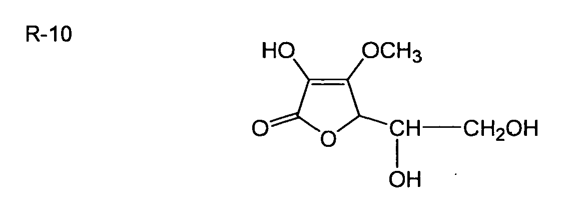

More preferred compounds as the reductones used as the component (b) of the hair dye of the present invention are reductones represented by the aforementioned general formula (I). Specific examples of preferred reductones are the reductones of the aforementioned Exemplary Compounds R-1 to R-8, and among them, ascorbic acid (Exemplary Compound R-2) is particularly preferred. As ascorbic acid, D-ascorbic acid, L-ascorbic acid or a mixture thereof can be used. Ascorbic acid is a natural product, and accordingly is particularly preferred from a viewpoints of cost and safety. Reductones can also be used in the form of an alkali metal salt such as lithium salt, sodium salt and potassium salt, ammonium salt, or the like.

Although the amount of the reductone used as the component (b) of the hair dye of the present invention is not particularly limited, when the solution B in the form of a solution containing the component (b) is prepared, the solution B preferably contains 0.05 to 20% by mass, most preferably 0.1 to 10% by mass, of the reductone. By altering concentration of the reductone of the component (b) of the hair dye of the present invention, the color tone of hair after hair dyeing can be changed.

Type of the carboxylic acid silver salt and/or the sulfonic acid silver salt used as the component (a) of the hair dye of the present invention is not particularly limited, and any of such silver salts having sufficient water-solubility may be used. Examples include, for example, a carboxylic acid silver salt represented by the following general formula (II): R¹-(COOAg)ₙ (wherein R¹ represents an n-valent organic group ; and n represents an integer of 1 to 3), a sulfonic acid silver salt represented by the following general formula (III) : R²-(SO₃Ag)ₙ (wherein R² represents an n-valent organic group, and n represents an integer of 1 to 3), and the like, but not limited to these examples. Two or more kinds of the carboxylic acid silver salts and/or the sulfonic acid silver salts may be used in combination. Further, the carboxylic acid silver salt and/or the sulfonic acid silver salt may be used in combination with another water-soluble silver salt. Examples of the other water-soluble silver salt include a silver ammonium complex salt, silver nitrate and silver sulfate. Since certain silver halides such as silver chloride and silver bromide are substantially water-insoluble, it is not desirable to use such halides as the component (a) of the hair dye of the present invention. Further, as the halides contain a halogen ion and may influence the environment, they are not preferred.

As the carboxylic acid silver salt and/or the sulfonic acid silver salt used as the component (a) of the present invention, a silver salt having a water solubility of 0.03% by mass or higher in terms of silver is preferred, and a silver salt having a water solubility of 0.3% by mass or higher is particularly preferred. When a solution A containing the component (a) of the hair dye is prepared in a liquid form, content of the carboxylic acid silver salt and/or the sulfonic acid silver salt silver is preferably 0.03 to 20% by mass, most preferably 0.03 to 10% by mass, in terms of silver. By altering concentration of the carboxylic acid silver salt and/or the sulfonic acid silver salt of the component (a) of the hair dye of the present invention, the color tone of hair after hair dyeing can be changed.

As the n-valent organic group represented by R¹ in the silver carboxylate represented by general formula (II), an organic group having 1 to 20 carbon atoms in total is preferred, an organic group having 1 to 6 carbon atoms in total is more preferred, and an organic group having 2 to 4 carbon atoms in total can be most preferably used. Symbol n is preferably 1 to 3, most preferably 1 or 2. The n-valent organic group represented by R¹ may be either an aliphatic organic group or an aromatic organic group, and an aliphatic organic group can be preferably used. The n-valent organic group represented by R¹ may have one or more of substituents. Examples of the substituents include, for example, hydroxyl group, an alkoxy group, an aryloxy group, an alkyl group, an aryl group, carboxy group, an alkoxycarbonyl group, an aryloxycarbonyl group, an alkoxycarbonyloxy group, amino group, amido group, a carbamoyl group, a carbamoylamino group, an alkoxycarbonylamino group, a carbamoyloxy group, an alkylsulfanyl group, an arylsulfanyl group, an alkylsulfonyl group, an arylsulfonyl group, sulfonamido group, sulfamoyl group, sulfo group, and the like, and particularly preferred examples of the substituents include hydroxyl group, an alkoxy group, an alkyl group, carboxy group, amino group, amido group, a carbamoyl group, an alkylsulfanyl group, an alkylsulfonyl group and sulfo group.

Examples of the aliphatic carboxylic acid constituting the silver carboxylate represented by the general formula (II) include, for example, acetic acid, propionic acid, butyric acid, isobutyric acid, valeric acid, trifluoroacetic acid, behenic acid, lactic acid, glycolic acid, levulinic acid, fumaric acid, maleic acid, succinic acid, glycine, α - alanine, β -alanine, valine, leucine, isoleucine, phenylalanine, proline, serine, threonine, tyrosine, aspartic acid, glutamic acid, arginine, lysine, histidine, tryptophan, cysteine, cystine, methionine, tartaric acid, malic acid, citric acid, mandelic acid, nicotinic acid, pyroglutamic acid, and the like. Among them, acetic acid, propionic acid, lactic acid, glycolic acid, pyroglutamic acid and proline are particularly preferred. Further, as the aromatic carboxylic acid constituting the silver carboxylate represented by the general formula (IV), for example, benzoic acid, salicylic acid, 4-hydroxybenzoic acid, 4-aminobenzoic acid, anthranilic acid, 4-amino-2-hydroxybenzoic acid, phthalic acid, terephthalic acid, trimellitic acid, pyromellitic acid, 2,6-naphthalenedicarboxylic acid and ortho-sulfobenzoic acid are preferred.

As the n-valent organic group represented by R² in the silver sulfonate represented by the general formula (III), an organic group having 1 to 20 carbon atoms in total is preferred, an organic group having 1 to 7 carbon atoms in total is more preferred, and an organic group having 1 to 4 carbon atoms in total is particularly preferred. Symbol n is preferably 1 to 3, most preferably 1 or 2. The n-valent organic group represented by R² may be either an aliphatic organic group or an aromatic organic group, and an aliphatic organic group can be preferably used. The n-valent organic group represented by R² may have one or more of substituents. Examples of the substituents include, for example, hydroxyl group, an alkoxy group, an aryloxy group, an alkyl group, an aryl group, carboxy group, an alkoxycarbonyl group, an aryloxycarbonyl group, an alkoxycarbonyloxy group, amino group, amido group, a carbamoyl group, a carbamoylamino group, an alkoxycarbonylamino group, a carbamoyloxy group, an alkylsulfanyl group, an arylsulfanyl group, an alkylsulfonyl group, an arylsulfonyl group, sulfonamido group, sulfamoyl group, sulfo group, and the like. Particularly preferred examples of the substituents include hydroxyl group, an alkoxy group, an alkyl group, carboxy group, amino group, amido group, a carbamoyl group, an alkylsulfanyl group, an alkylsulfonyl group, sulfo group, and the like.

As the aliphatic sulfonic acid constituting the silver sulfonate represented by general formula (III), for example, methanesulfonic acid, ethanesulfonic acid, propanesulfonic acid, butanesulfonic acid, hexanesulfonic acid, taurine, hydroxyethanesulfonic acid, trifluoromethanesulfonic acid and 1,4-butanedisulfonic acid are preferred, and methanesulfonic acid and ethanesulfonic acid are particularly preferred.

As the aromatic sulfonic acid constituting the silver sulfonate represented by the general formula (III), for example, benzenesulfonic acid, 4-toluenesulfonic acid, 4-chlorobenzenesulfonic acid, 4-hydroxybenzenesulfonic acid, orthanilic acid, metanilic acid, sulfanilic acid, ortho-sulfobenzoic acid, 1,5-naphthalenedisulfonic acid, and the like are preferred.

The component (b) of the hair dye of the present invention may contain a reducing agent other than reductones. Examples include, for example, paraphenylenediamines (specifically, para-phenylenediamine, 2,5-diaminotoluene, 4,4'-diaminodiphenylamine, 2-chloro-para-phenylenediamine, N-phenyl-para-phenylenediamine, salts thereof, and the like), ortho-phenylenediamines (specifically, ortho-phenylenediamine, salts thereof, and the like), para-aminophenols (specifically, para-aminophenol, salts thereof, and the like), ortho-aminophenols (specifically, ortho-aminophenol, 2,4-diaminophenol, salts thereof, and the like), hydroquinones (specifically, hydroquinone, 2-sulfohydroquinone, salts thereof, and the like), and catechols (specifically, catechol, pyrogallol, gallic acid, methyl gallate, 1,2,4-benzenetriol, salts thereof, and the like). Two or more kinds of reducing agents other than reductones may be used in combination. Among them, para-phenylenediamine, 2,5-diaminotoluene, para-aminophenol, ortho-aminophenol, and 4,4'-diaminodiphenylamine are particularly preferred.

A surfactant may be mixed in the component (a) and/or the component (b) of the hair dye of the present invention. Specific examples of the surfactant include, for example, anionic surfactants, cationic surfactants, nonionic surfactant and amphoteric surfactants, and two or more types of these surfactants may be used in combination.

Examples of the anionic surfactants include alkylsulfates such as sodium laurylsulfate, salts of alkylbenzenesulfonic acids such as dodecylbenzenesulfonic acid, alkylnaphthalenesulfonates, alkylsulfonates such as sodium tetradecenesulfonate, and the like.

Examples of the cationic surfactants include quaternary ammonium salts such as cetyltrimethylammonium chloride, stearyltrimethylammonium chloride, lauryltrimethylammonium chloride, distearyldimethylammonium chloride, cetyltrimethylammonium bromide, stearyltrimethylammonium bromide, lauryltrimethylammonium bromide, distearyldimethylammonium bromide and cetylpyridinium bromide.

Examples of the nonionic surfactants include polyethylene oxides, polyethylene oxide monoalkyl ethers, polyethylene oxide diethers, polyethylene oxide monoalkenyl ethers, polyethylene oxide monophenyl ethers, polypropylene oxides, polypropylene oxide monoalkyl ethers, polypropylene oxide dialkyl ethers, polyethylene oxide fatty acid esters, glycerin fatty acid esters, glycerin monoethers (monostearyl glyceryl ether, monocetyl glyceryl ether, monooleyl glyceryl ether, isostearyl glyceryl ether and the like) and sorbitan fatty acid esters. Among them, preferred are polyethylene oxides, polyethylene oxide monoalkyl ethers, polyethylene oxide diethers, polyethylene oxide monoalkenyl ethers, polyethylene oxide monophenyl ethers, polypropylene oxides, polypropylene oxide monoalkyl ethers and polypropylene oxide dialkyl ethers, and particularly preferred are polyethylene oxides, polyethylene oxide monoalkyl ethers, polyethylene oxide diethers, polyethylene oxide monoalkenyl ethers, polypropylene oxides and polypropylene oxide monoalkyl ethers.

Examples of the amphoteric surfactants include betaine lauryldimethylaminoacetate, betaine cetyldimethylaminoacetate, betaine stearyldimethylaminoacetate, and the like.

These surfactants may be independently used, or two or more types of the surfactants may be used in combination. These surfactants are used preferably in the range of 0.5 to 55% by mass, desirably in the range of 2 to 50% by mass, based on the total mass of the hair dye upon use.

An oily component may also be mixed in the component (a) and/or the component (b) of the hair dye of the present invention, preferably the solution A and/or solution B in the form of liquid. Type of the oily component is not particularly limited so far that the oily component does not degrade the performance of the hair dye and can be dissolved or dispersed in the hair dye. By mixing the oily component, damage to hair after hair dyeing can be suppressed to allow the hair to have gloss and moisture. Examples of the oily component include silicones, hydrocarbons, oils and fats, waxes, higher fatty acids, and the like.

Examples of silicone derivatives include, for example, dimethylpolysiloxane, methylphenylpolysiloxane, decamethylcyclopentasiloxane, dodecamethylcyclohexasiloxane, polyether-denatured silicone, highly polymerized silicone, amino-denatured silicone, betaine-denatured silicone, alkyl-denatured silicone, mercapto-denatured silicone, carboxy-denatured silicone, fluorine-denatured silicone, polyglycerin-denatured silicone (for example, those described in Japanese Patent Unexamined Publication Nos. 10-316540 and 2006-69893), and the like.

Examples of the hydrocarbons include squalanes, α -olefin oligomers, paraffins, isoparaffins, vaseline, and the like.

Examples of the oils and fats include olive oil, camellia oil, tea seed oil, safflower oil, sunflower oil, soybean oil, cotton seed oil, sesame oil, cacao butter, corn oil, peanut oil, rape seed oil, and the like.

Examples of the waxes include beeswax, carnauba wax, jojoba oil, lanolin, and the like.

Examples of the higher fatty acids include lauric acid, myristic acid, palmitic acid, stearic acid, behenic acid, oleic acid, stearic acid, hydroxystearic acid, linoleic acid, and the like.

These oily components may be independently used, or two or more types of them may be used in combination. Although mixing amount of the oily component is not particularly limited, the amount is, for example, preferably 0.005 to 10% by mass, more preferably 0.01 to 5% by mass, still more preferably 0.05 to 2% by mass, based on the total mass of the hair dye upon use.

An alcohol may be added to the component (a) and/or the component (b) of the hair dye of the present invention, preferably the solution A and/or solution B in the form of liquid. Type of the alcohol is not particularly limited so far that the alcohol does not degrade the performance of the hair dye and can be dissolved or dispersed in the hair dye. Examples of the alcohol include, for example, cetanol, oleyl alcohol, stearyl alcohol and hexadecanol. These alcohols may be independently used, or two or more types of alcohols may be used in combination. Although mixing amount of the alcohol is not particularly limited, the amount is, for example, preferably 0.005 to 10% by mass, more preferably 0.01 to 5% by mass, still more preferably 0.05 to 2% by mass, based on the total mass of the hair dye upon use.

A polymer compound may be added to the component (a) and/or the component (b) of the hair dye of the present invention. Type of the polymer compound is not particularly limited so far that the polymer compound does not degrade the performance of the hair dye and can be dissolved or dispersed in the hair dye. Examples of the polymer compound include, for example, cationic polymer compounds, amphoteric polymer compounds, anionic polymer compounds and nonionic polymer compounds.

Examples of the cationic polymer compounds include cationized cellulose (for example, O-[2-hydroxy-3-(trimethylammonio)propyl]hydroxyethylcellulose chloride, O-[2-hydroxy-3-(lauryldimethylammonio)propyl)hydroxyethylcellulose) chloride, cationic starch, cationized guar gum derivatives (for example, the derivatives described in Japanese Patent Publication Nos. 58-35640 and 60-46158 and Japanese Patent Unexamined Publication No. 58-53996), and polydimethyldiallylammonium salts.

Examples of the anionic polymer compounds include, for example, copolymers of acrylic acid or methacrylic acid and other vinyl monomers, and carboxymethylcellulose.

Examples of the nonionic polymer compounds include, for example, polyvinyl alcohol, polyvinylpyrrolidone, cellulose, methylcellulose, ethylcellulose, hydroxyethylcellulose, hydroxypropylcellulose, hydroxypropylmethylcellulose, guar gum, hydroxypropyl xanthan gum, agar, starch, polyacrylic acid ester, polymethacrylic acid ester, polyacrylamide, polymethacrylamide, polyethylene glycol and polypropylene glycol.

Examples of other useful polymer compounds include, for example, natural polymer compounds such as gum arabic, carrageenan, galactan, quince seed gum, locust bean gum, tragacanth gum, pectin, mannan, xanthan gum, dextran, curdlan, gellan gum, succinoglucan, gelatin, tamarind gum and casein.

These polymer compounds may be independently used, or two or more types of the polymers may be used in combination. Although mixing amount of the polymer compound is not particularly limited, the amount is, for example, 0.01 to 10% by mass, preferably 0.1 to 5% by mass, based on the total mass of the hair dye upon use.

A thickener may be mixed in the component (a) and/or the component (b) of the hair dye of the present invention. Examples of the thickener include, for example, sodium alginate, gum arabic, crosslinked acrylic acid polymers, cellulose derivatives, xanthan gum, bentonite, and the like. These thickeners may be independently used, or two or more types thereof may be used in combination. Although mixing amount of the thickener is not particularly limited, the amount is, for example, preferably 0.1 to 5% by mass, most preferably 0.2 to 3% by mass, based on the total mass of the hair dye upon use.

In order to adjust the color tone, a known direct dye can be mixed in the component (a) and/or the component (b) of the hair dye of the present invention. Examples of the known direct dye include, for example, Black No. 401, Blue No. 1, Blue No. 2, Blue No. 201, Blue No. 202, Blue No. 203, Blue No. 204, Blue No. 205, Blue No. 403, Blue No. 404, Purple No. 201, Purple No. 401, Red No. 102, Red No. 106, Red No. 201, Red No. 202, Red No. 203, Red No. 204, Red No. 205, Red No. 206, Red No. 207, Red No. 208, Red No. 213, Red No. 214, Red No. 215, Red No. 218, Red No. 219, Red No. 220, Red No. 221, Red No. 223, Red No. 225, Red No. 226, Red No. 227, Orange No. 201, Orange No. 203, Orange No. 205, Orange No. 206, Orange No. 207, Orange No. 401, Orange No. 402, Orange No. 403, Yellow No. 201, Yellow No. 203, Yellow No. 204, Yellow No. 205, Yellow No. 206, Yellow No. 207, Yellow No. 401, Yellow No. 402, Yellow No. 403, and the like.

To the component (a) and/or the component (b) of the hair dye of the present invention, an autoxidation dye, of which typical examples include indoles, indolines and the like, or a known direct dye such as nitro dyes and disperse dyes may also be added.

Other substances may be further added to the component (a) and/or the component (b) of the hair dye of the present invention. Types of the other substances are not particularly limited so far that the substance does not degrade the performance of the hair dye and can be dissolved or dispersed in the hair dye. Examples of the other substances include, for example, collagen, keratin, elastin, fibroin, conchiolin, sodium pyrrolidonecarboxylate, sodium lactate, sorbitol, hyaluronic acid, benzyl alcohol, phenethyl alcohol, benzyloxyethanol, N-methylpyrrolidone, N-ethylpyrrolidone, ethylene carbonate, propylene carbonate, paraben, ultraviolet absorbers, and the like. pH of the hair dye can be appropriately adjusted. In view of hair dyeing property, pH of a liquid composition obtained by mixing the component (a) and the component (b), preferably mixing the solution A and the solution B, is preferably in the range of 2 to 11, most preferably in the range of 2 to 8. From a viewpoint of suppression of damage to hair, pH of the aforementioned liquid composition is preferably 8 or lower, more preferably 7 or lower, most preferably in the range of 5 to 7. From a viewpoint of storage stability of the hair dye of the present invention, pH of the solution A is preferably in the range of 3 to 7, most preferably in the range of 5 to 7. pH of the solution B of the hair dye of the present invention is preferably in the range of 3 to 7, more preferably in the range of 5 to 7. Although pH of the hair dye may be unadjusted, it is also preferable to adjust the pH by using an appropriate pH modifier.

As the pH modifier used to adjust pH of the component (a) and/or the component (b) of the hair dye of the present invention, suitable acids can be used. More specifically, examples include organic acids such as acetic acid, lactic acid, tartaric acid, malic acid, citric acid, glycolic acid, pyrrolidonecarboxylic acid, levulinic acid, fumaric acid, succinic acid, butyric acid, valeric acid, oxalic acid, maleic acid, mandelic acid, aspartic acid, adipic acid and nicotinic acid, and inorganic acids such as phosphoric acid, sulfuric acid and nitric acid. Among them, preferably used acids include organic acids such as acetic acid, lactic acid, tartaric acid, malic acid, citric acid, glycolic acid, fumaric acid, succinic acid, butyric acid, valeric acid, maleic acid, mandelic acid, aspartic acid and adipic acid. Salts of these acids (sodium salts, potassium salts, ammonium salts etc.) may also be used as the pH modifier. From a viewpoint of stability of the solution A and suppression of damage to hair, organic acids are preferably used as the pH modifier.

As the pH modifier used to adjust pH of the component (a) and/or the component (b) of the hair dye of the present invention, appropriate bases may also be used. More specifically, examples include, for example, alkali metal hydroxide such as sodium hydroxide and potassium hydroxide, alkali metal carbonates such as sodium carbonate and potassium carbonate, ammonia, and organic bases such as ethanolamine, diethanolamine and guanidine. Salts of these bases (hydrochlorides, sulfates, and the like) may also be used as the pH modifier.

Although content of the pH modifier is not particularly limited, the content is, for example, preferably 0.01 to 20% by mass, more preferably 0.1 to 10% by mass, most preferably 0.5 to 5% by mass, based on the total mass of the hair dye upon use.

A stabilizer may be added to the component (a), preferably to the solution A, of the hair dye of the present invention to improve stability of the silver salt. Examples of the stabilizer include, for example, sulfur. Although amount of sulfur to be added is not particularly limited, the amount is, for example, 0.01 to 1 % by weight based on the total mass of the component (a).

An aromatic may be added to the component (a) and/or the component (b) of the hair dye of the present invention, as required.

The hair dye of the present invention can be used to dye human or animal hair, for example, to dye hair, body hair, animal body hair, and the like, preferably human hair and body hair of pets such as dogs and cats, most preferably human hair.

The component (a) and/or the component (b) of the hair dye of the present invention can be provided, for example, in a form separately comprising the components, for example, separately packaged dry solids of the components, or in a form comprising both the component (a) and the component (b), for example, a dry mixture thereof. When the hair dye of the present invention is provided as a dry solid as described above, the component (a) and the component (b) or a mixture thereof can be dissolved or suspended before use in, for example, water, ethanol, isopropanol, ethylene glycol, diethylene glycol, propylene glycol, dipropylene glycol, 1,3-butylene glycol, isoprene glycol, hexylene glycol, ethyl carbitol, glycerin, diglycerin, polyethylene glycol, polypropylene glycol, mixture thereof or the like, and used as the hair dye.

Further, the hair dye of the present invention can also be provided in a liquid form separately comprising the component (a) and the component (b), or a liquid form comprising a mixture thereof. In the specification, the term "solution A" means a liquid containing the component (a), and the term "solution B" means a liquid containing the component (b). In the specification, the term "liquid form" means a form other than solid and gas, and examples thereof include forms of solution, suspension, emulsion, cream, foam, gel, paste and the like. Although type of the solvent for providing the hair dye in a liquid form is not particularly limited, for example, water, methanol, isopropanol, ethylene glycol, diethylene glycol, propylene glycol, dipropylene glycol, 1,3-butylene glycol, isoprene glycol, hexylene glycol, ethyl carbitol, glycerin, diglycerin, polyethylene glycol, polypropylene glycol, and the like can be used. These solvents may be used alone, or two or more kinds of the solvents may be used in combination. It is preferable to use water alone, or a mixture of water and any of the aforementioned organic solvents. Among these forms, preferred is a form of solution, and it is most preferable to provide the component (a) and the component (b) in the forms of solutions separately filled in different containers. However, the hair dye of the present invention can be prepared in an arbitrary form suitable for hair dyeing. For example, the hair dye of the present invention can also be provided in a form that the component (a) and/or the component (b), or a mixture thereof is filled in an aerosol container with pressurization together with a propellant, or the like.

When hair is dyed by using the hair dye of the present invention, the order of application of the component (a) and the component (b) is not particularly limited, and they can be applied in an arbitrary order. For example, when hair is dyed by using the solution A and the solution B in liquid forms, the solution A may be applied to hair first, and then the solution B may be applied, or the solution B of the hair dye may be applied to hair first, and then the solution A may be applied. Alternatively, the hair dye compositions, the solution A and the solution B, may be mixed immediately before use, and the resulting mixture may be applied. Among these methods, the method of applying the solution A, and then applying the solution B, and the method of mixing the dye compositions, the solution A and the solution B, immediately before use and applying the mixture are preferred. The method of applying the solution A, and then applying the solution B is particularly preferred. After the solution A is applied and left for a while, then solution B may be applied and further left for a while. Although a period of time for the leaving is not particularly limited, the period is, for example, preferably 0 to 30 minutes, most preferably 5 to 20 minutes. Similarly, after the hair dye solution B is applied and left for a while, and then the solution A may be applied and further left for a while. Although a period of time for the leaving is not particularly limited, the period is, for example, preferably 0 to 30 minutes, most preferably 5 to 20 minutes. The hair dyeing treatment can be performed at, for example, a temperature of about 0 to 40°C. Although amounts of the solution A and the solution B to be used are not particularly limited, they are preferably used in a volume ratio in the range of 3/1 to 1/3. The method for applying the solution A and/or the solution B or a mixture thereof is not particularly limited, and an arbitrary method such as a method of applying the solutions to hair, or a method of immersing hair in the solutions can be adopted. In a hair dyeing method using the hair dye of the present invention, hair can be treated with shampoo and/or conditioner after the hair dyeing. Then, the hair is preferably dried as required.

### Examples

The present invention will be explained more specifically with reference to the following examples. However, the scope of the present invention is not limited by these examples.

### Example 1

Silver lactate 0.5-hydrate was weighed in an amount of 0.195 g, added with ion-exchanged water to make the total weight 22.0 g, and dissolved by stirring to obtain a solution A of the hair dye (0.46% by mass in terms of silver). pH of the solution A was 7. L-Ascorbic acid was weighed in amount of 1.091 g, added with ion-exchanged water to make the total weight 72.0 g, and dissolved by stirring to obtain a solution B of the hair dye (L-ascorbic acid concentration : 1.5% by mass). pH of the solution B was 2.63. The solution A obtained above was applied to white goat hair, and the hair was left at 30°C for 20 minutes. The solution B obtained above was applied to the goat hair, and the hair was left at 30°C for 20 minutes, washed with warm water, and dried by using a drier. Hair dyeing density (Δ E) of the dyed goat hair was measured by using a chroma meter (CR-400, Konica Minolta Sensing, Inc.). Further, damage to the goat hair after the hair dyeing was evaluated on the basis of touch.

### Example 2

Goat hair was dyed and evaluated in the same manner as that in Example 1 except that 0.158 g of silver acetate was used instead of 0.195 g of silver lactate 0.5-hydrate (silver concentration of the solution A was 0.46% by mass).

### Example 3

Goat hair was dyed and evaluated in the same manner as that in Example 1 except that 0.162 g of silver citrate was used instead of 0.195 g of silver lactate 0.5-hydrate (silver concentration of the solution A was 0.46% by mass). However, a part of silver citrate was not dissolved in this experiment.

### Example 4

Goat hair was dyed and evaluated in the same manner as that in Example 1 except that 0.192 g of silver methanesulfonate was used instead of 0.195 g of silver lactate 0.5-hydrate (silver concentration of the solution A was 0.46% by mass).

### Example 5

Goat hair was dyed and evaluated in the same manner as that in Example 1 except that 0.246 g of silver para-toluenesulfonate was used instead of 0.195 g of silver lactate 0.5-hydrate (silver concentration of the solution A was 0.46% by mass).

### Comparative Example 1

Goat hair was dyed and evaluated in the same manner as that in Example 1 except that 0.148 g of silver sulfate was used instead of 0.195 g of silver lactate 0.5-hydrate (silver concentration of the solution A was 0.46% by mass). However, a part of silver sulfate was not dissolved in this experiment.

### Comparative Example 2

Goat hair was dyed and evaluated in the same manner as that in Example 1 except that 0.161 g of silver nitrate was used instead of 0.195 g of silver lactate 0.5-hydrate (silver concentration of the solution A was 0.46% by mass).

### Comparative Example 3

Goat hair was dyed and evaluated in the same manner as that in Example 1 except that 0.161 g of silver nitrate and 1.0 mL of 28% aqueous ammonia were used instead of 0.195 g of silver lactate 0.5-hydrate (silver concentration of the solution A was 0.46% by mass, pH of the solution A was 10, and silver ammonium complex salt was produced in the solution A). During the hair dyeing, unpleasant odor emanated when the solution A was applied.

### Comparative Example 4

Goat hair was dyed and evaluated in the same manner as that in Example 1 except that 0.136 g of silver chloride was used instead of 0.195 g of silver lactate 0.5-hydrate (silver concentration of the solution A was 0.46% by mass). However, silver chloride was hardly dissolved in this experiment.

### Comparative Example 5

Goat hair was dyed and evaluated in the same manner as that in Example 1 except that 0.178 g of silver bromide was used instead of 0.195 g of silver lactate 0.5-hydrate (silver concentration of the solution A was 0.46% by mass). However, silver bromide was hardly dissolved in this experiment.

### Comparative Example 6

Goat hair was dyed and evaluated in the same manner as that in Example 1 except that 0.161 g of silver nitrate and 1.0 mL of 28% aqueous ammonia were used instead of 0.195 g of silver lactate 0.5-hydrate, and 0.670 g of para-phenylenediamine (PPD) was used instead of 1.091 g of L-ascorbic acid (silver concentration of the solution A was 0.46% by mass, pH of the solution A was 10, silver ammonium complex salt was produced in the solution A, and pH of the solution B was 9.29). During the hair dyeing, unpleasant odor emanated when the solution A was applied.

### Comparative Example 7

Goat hair was dyed and evaluated in the same manner as that in Example 1 except that 0.161 g of silver nitrate and 1.0 mL of 28% aqueous ammonia were used instead of 0.195 g of silver lactate 0.5-hydrate, and 1.409 g of 2-sulfohydroquinone potassium salt (HQ-SK) and 1.1 mL of 1 N aqueous sodium hydroxide were used instead of 1.091 g of L-ascorbic acid (silver concentration of the solution A was 0.46% by mass, pH of the solution A was 10, silver ammonium complex salt was produced in the solution A, and pH of the solution B was 9.47). During the hair dyeing, unpleasant odor emanated when the solution A was applied.

The evaluation results are shown in Table 1 mentioned below. From these results, it can be understood that the hair dye of the present invention has superior hair dyeing property, and does not cause significant damage to hair. In contrast, it was found that hair was not dyed when a substantially water-insoluble silver halide was used, and that unpleasant odor emanated during hair dyeing, and hair was damaged as well when a silver ammonium complex salt was used.

**Table 1**

| | Silver salt | Reducing agent | Δ E | Damage to goat hair |
|---|---|---|---|---|
| Example 1 | Silver lactate | Ascorbic acid | 46.5 | ○ |
| Example 2 | Silver acetate | Ascorbic acid | 51.8 | ○ |
| Example 3 | Silver citrate | Ascorbic acid | 42.4 | ○ |
| Example 4 | Silver methanesulfonate | Ascorbic acid | 49.2 | ○ |
| Example 5 | Silver para-toluenesulfonate | Ascorbic acid | 48.8 | ○ |
| Comparative Example 1 | Silver sulfate | Ascorbic acid | 47.1 | ○ |
| Comparative Example 2 | Silver nitrate | Ascorbic acid | 50.0 | ○ |
| Comparative Example 3 | Silver ammonium complex salt | Ascorbic acid | 49.6 | Δ |
| Comparative Example 4 | Silver chloride | Ascorbic acid | 3.4 | ○ |
| Comparative Example 5 | Silver bromide | Ascorbic acid | 3.7 | ○ |
| Comparative Example 6 | Silver ammonium complex salt | PPD | 54.7 | × |
| Comparative Example 7 | Silver ammonium complex salt | HQ-SK | 36.0 | × |

| | | | | |
|---|---|---|---|---|
| Damage to goat hair ○ : Touch is identical to touch before hair dyeing. Δ : Touch is degraded compared with that before hair dyeing. × : Touch is markedly degraded compared with that before hair dyeing. | | | | |

### Example 6

Silver lactate 0.5-hydrate was weighed in an amount of 0.016 g, added with ion-exchanged water to make the total weight 15.0 g, and dissolved by stirring to obtain a solution A of the hair dye. L-Ascorbic acid was weighed in amount of 2.417 g, added with ion-exchanged water to make the total weight 60.0 g, and dissolved by stirring to obtain a solution B of the hair dye. The solution A obtained above was applied to white goat hair, and the hair was left at 30°C for 20 minutes. The solution B obtained above was applied to the goat hair, and the hair was left at 30°C for 20 minutes, washed with warm water, and dried by using a drier. Hair dyeing density (Δ E) of the dyed goat hair was measured by using a chroma meter (CR-400, Konica Minolta Sensing, Inc.). Further, damage to the goat hair after the hair dyeing was evaluated on the basis of touch.

### Example 7

Goat hair was dyed and evaluated in the same manner as that in Example 6 except that 0.078 g of silver lactate 0.5-hydrate was used instead of 0.016 g of silver lactate 0.5-hydrate.

### Example 8

Goat hair was dyed and evaluated in the same manner as that in Example 6 except that 0.136 g of silver lactate 0.5-hydrate was used instead of 0.016 g of silver lactate 0.5-hydrate.

### Example 9

Goat hair was dyed and evaluated in the same manner as that in Example 6 except that 0.470 g of silver lactate 0.5-hydrate was used instead of 0.016 g of silver lactate 0.5-hydrate.

The results are shown in Table 2 mentioned below. It can be understood that various color tones and densities can be expressed by adjusting the concentration of the silver salt in the hair dye of the present invention.

**Table 2**

| | Silver concentration | Δ E | Color tone | Damage to goat hair |
|---|---|---|---|---|
| Example 6 | 0.06% | 32.2 | Yellow | ○ |
| Example 7 | 0.27% | 45.3 | Yellowish brown | ○ |
| Example 8 | 0.47% | 48.1 | Reddish brown | ○ |
| Example 9 | 1.64% | 51.5 | Black | ○ |

| | | | | |
|---|---|---|---|---|
| Damage to goat hair ○ : Touch is identical to touch before hair dyeing. Δ : Touch is slightly degraded compared with that before hair dyeing. × : Touch is clearly degraded compared with that before hair dyeing. | | | | |

### Examples 10 to 12

Shampoo resistance and conditioner resistance of dyed goat hairs obtained in the same manners as those of Examples 6, 8 and 9 were investigated. The example numbers corresponding to the experiments using the dyed goat hairs obtained in the same manners as those of Examples 6, 8 and 9 are Examples 10, 11 and 12, respectively. Each dyed goat hair was wetted with warm water, washed with shampoo, then washed with warm water, further treated with conditioner, and then washed with warm water. These washing with shampoo and treatment with conditioner were repeated 40 times. After the hair was dried, the density was measured. The results are shown in Table 3 mentioned below. The hairs dyed with the hair dyes of the present invention showed small color difference between colors before and after the shampoo and conditioning, and thus it can be understood that the hair dye of the present invention provides superior shampoo resistance and conditioner resistance.

**Table 3**

| Goat hair used | 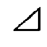 E before shampoo and conditioning | 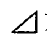 E after shampoo and conditioning |
|---|---|---|
| Example 10 | 34.8 | 38.8 |
| Example 11 | 48.5 | 49.1 |
| Example 12 | 52.6 | 52.5 |

### Examples 13 to 15

Light resistance of dyed goat hairs obtained in the same manners as those of Examples 6, 8 and 9 were investigated. The example numbers corresponding to the experiments using the dyed goat hairs obtained in the same manners as those of Examples 6, 8 and 9 are Examples 13, 14 and 15, respectively. Each dyed hair was dried, and illuminated with light from a xenon lamp of 170,000 luxes for 93 hours, and then the density was measured. The results are shown in Table 4 mentioned below. The hairs dyed with the hair dyes of the present invention gave small color difference between colors before and after the irradiation of light from a xenon lamp, and thus it can be understood that the hair dye of the present invention provides superior light resistance.

**Table 4**

| Goat hair used | 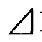 E before light resistance test | 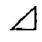 E after light resistance test |
|---|---|---|
| Example 13 | 30.9 | 33.3 |
| Example 14 | 49.1 | 52.3 |
| Example 15 | 51.8 | 54.3 |

### Example 16

Silver nitrate and sodium glycolate were weighed in amounts of 0.0878 g and 0.0517 g, respectively, added with ion-exchanged water to make the total weight 12.0 g, and dissolved by stirring to obtain a solution A of the hair dye composition of the present invention (0.46% by mass in terms of silver). L-Ascorbic acid was weighed in amount of 0.4993 g, added with ion-exchanged water to make the total weight 33.0 g, and dissolved by stirring to obtain a solution B of the hair dye composition of the present invention (L-ascorbic acid concentration: 1.51% by mass). The solution A obtained above was applied to white goat hair, and the hair was left for 20 minutes. The solution B obtained above was applied to the goat hair, and the hair was left for 20 minutes, washed with warm water, and dried by using a drier. Hair dyeing density (Δ E) of the dyed goat hair was measured by using a chroma meter (CR-400, Konica Minolta Sensing, Inc.).

### Comparative Example 8

Goat hair was dyed and evaluated in the same manner as that in Example 16 except that 0.0872 g of silver nitrate was used instead of the mixture of 0.0878 g of silver nitrate and 0.0517 g of sodium glycolate (silver concentration of the solution A was 0.46% by mass).

### Example 17

Silver nitrate and proline were weighed in amounts of 0.4276 g and 0.2883 g, respectively, as a mixture, added with ion-exchanged water to make the total weight 25.0 g, and dissolved by stirring to obtain a solution A of the hair dye composition of the present invention (1.08% by mass in terms of silver). L-Ascorbic acid was weighed in amount of 1.3227 g, added with ion-exchanged water to make the total weight 25.0 g, and dissolved by stirring to obtain a solution B of the hair dye composition of the present invention (L-ascorbic acid concentration: 5.27% by mass). The solution A obtained above was applied to white goat hair, and the hair was left for 20 minutes. The solution B obtained above was applied to the goat hair, and the hair was left for 20 minutes, washed with warm water, and dried by using a drier. Hair dyeing density (ΔE) of the dyed goat hair was measured by using a chroma meter (CR-400, Konica Minolta Sensing, Inc.).

### Example 18

Goat hair was dyed and evaluated in the same manner as that in Example 17 except that a mixture of 0.4279 g of silver nitrate, 0.3237 g of DL-pyroglutamic acid and 0.1014 g of sodium hydroxide was used instead of the mixture of 0.4276 g of silver nitrate and 0.2883 g of proline (silver concentration of the solution A was 1.08% by mass).

### Comparative Example 9

Goat hair was dyed and evaluated in the same manner as that in Example 17 except that 0.4270 g of silver nitrate was used instead of the mixture of 0.4276 g of silver nitrate and 0.2883 g of proline (silver concentration of the solution A was 1.08% by mass).

The results are shown in Table 5 mentioned below. It was found that high hair dyeing densities were obtainable with the hair dyes of the present invention using silver glycolate, proline silver salt and silver pyroglutamate.

**Table 5**

| | | |
|---|---|---|
| | Silver salt | Δ E |
| Example 16 | Silver glycolate | 47.6 |
| Comparative Example 8 | Silver nitrate | 49.6 |
| Example 17 | Proline silver salt | 53.5 |
| Example 18 | Silver pyroglutamate | 51.7 |
| Comparative Example 9 | Silver nitrate | 49.9 |

### Example 19

The solution A of the hair dye obtained in Example 1 in a volume of 0.5 mL was put on shaved skin of a rat. The solution was left for 10 minutes under illumination from an indoor fluorescent light, and density of the solution was confirmed by visual inspection. As a result, it was confirmed that a brown color was developed. Then, the skin was washed with soap. The coloring in brown was almost eliminated.

### Example 20

The same procedure as that of Example 19 was repeated except that the solution A of the hair dye obtained in Example 2 was used instead of the solution A of the hair dye obtained in Example 1. After illumination from a fluorescent light for 10 minutes, it was confirmed that a brown color was slightly developed. After washing of the skin with soap, the coloring in brown was eliminated.

### Comparative Example 10

The same procedure as that of Example 19 was repeated except that the solution A of the hair dye obtained in Comparative Example 1 was used instead of the solution A of the hair dye obtained in Example 1. After illumination from a fluorescent light for 10 minutes, it was confirmed that a brown color was developed. Even after washing of the skin with soap, the coloring was not eliminated.

### Comparative Example 11

The same procedure as that of Example 19 was repeated except that the solution A of the hair dye obtained in Comparative Example 2 was used instead of the solution A of the hair dye obtained in Example 1. After illumination from a fluorescent light for 10 minutes, it was confirmed that a black color was developed. Even after washing of the skin with soap, the coloring was not eliminated.

From the above results, it can be undetstood that the hair dye of the present invention has superior hair dyeing property for hair, whilst the dyeing ability thereof for the skin was weak. It was also found that, in contrast, when silver sulfate or silver nitrate was used, the dyeing ability thereof for the skin was high, and stains by coloring were caused.

## Claims

1. A hair dye comprising, together or separately:
(a) a carboxylic acid silver salt and/or a sulfonic acid silver salt, and
(b) a reductone.

2. The hair dye according to claim 1, wherein the reductone of the component (b) is a compound represented by the following general formula (I) or a salt thereof: 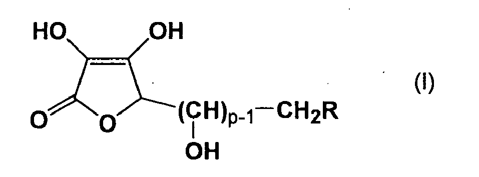 wherein R represents hydrogen atom or hydroxyl group; and p represents an integer of 1 to 4.

3. The hair dye according to claim 1 or 2, wherein the reductone is ascorbic acid or a salt thereof.

4. The hair dye according to any one of claims 1 to 3, wherein the carboxylic acid silver salt and/or the sulfonic acid silver salt of the component (a) is a silver salt selected from the group consisting of a carboxylic acid silver salt represented by the following general formula (II): R¹-(COOAg)ₙ wherein R¹ represents an n-valent organic group; and n represents an integer of 1 to 3, and a sulfonic acid silver salt represented by the following general formula (III): R²-(SO₃Ag)ₙ wherein R² represents an n-valent organic group, and n represents an integer of 1 to 3.

5. The hair dye according to claim 4, wherein the carboxylic acid silver salt and/or the sulfonic acid silver salt of the component (a) consists of one or more kinds of silver salts selected from the group consisting of silver acetate, silver lactate, silver glycolate, silver pyroglutamate, proline silver salt and silver methanesulfonate.

6. The hair dye according to any one of claims 1 to 5, which comprises two of solutions, a solution A containing a carboxylic acid silver salt and/or a sulfonic acid silver salt of the component (a) and a solution B containing a reductone of the component (b).

7. The hair dye according to claim 6, wherein content of the silver salt in the solution A is 0.01 to 10% by mass in terms of mass of silver based on the total mass of the solution A.

8. The hair dye according to claim 6 or 7, wherein content of the reductone in the solution B is 0.1 to 10% by mass based on the total mass of the solution B.

9. The hair dye according to any one of claims 6 to 8, wherein pH of the solution A and/or the solution B is 7 or lower.

10. A hair dyeing method comprising the step of applying a hair dye comprising (a) a carboxylic acid silver salt and/or a sulfonic acid silver salt, and (b) a reductone, together or separately, to hair.

11. A hair dyeing method using a hair dye comprising a combination of a solution A containing the component (a) and a solution B containing the component (b), which comprises the step of applying the solution A to hair and the step of applying the solution B to the hair in this order.
